Europäisches Patentamt

European Patent Office

Office européen des brevets

(19)

(11) Numéro de publication: **0 180 486**
**A1**

# (12) DEMANDE DE BREVET EUROPEEN

(21) Numéro de dépôt: 85401600.3

(22) Date de dépôt: 06.08.85

(51) Int. Cl.⁴: **C 12 N 5/00**, C 12 P 21/00, G 01 N 33/577, A 61 K 39/395

(30) Priorité: 06.08.84 FR 8412415

(43) Date de publication de la demande: 07.05.86
Bulletin 86/19

(84) Etats contractants désignés: **AT BE CH DE GB IT LI LU NL SE**

(71) Demandeur: **Etablissement Public dit: CENTRE NATIONAL DE LA RECHERCHE SCIENTIFIQUE (CNRS), 15, Quai Anatole France, F-75700 Paris (FR)**

(72) Inventeur: **Strosberg, Arthur, 66, rue de Javel, F-75015 Paris (FR)**
Inventeur: **Guillet, Jean-Gérard, 2, rue de la Pierre levée, F-75011 Paris (FR)**
Inventeur: **Hoebeke, Johan, 28, rue des Pierrelais, F-92320 Chatillon S/Bagneux (FR)**

(74) Mandataire: **Nony, Michel et al, Cabinet Nony 29, rue Cambacérès, F-75008 Paris (FR)**

(54) **Nouvelles cellules hybrides sécrétant des anticorps anti-idiotypiques, nouveaux anticorps monoclonaux sécrétés par lesdites cellules hybrides, leur préparation et leur application.**

(57) Lignées cellulaires hybdrides capables de sécréter des anticorps anti-idiotypiques dirigés contre une immunoglobuline anti-ligand; ces lignées cellulaires obtenues par fusion sont sélectionnées successivement selon les critères cumulatifs suivants:

a) les anticorps qu'elles sécrètent sont capables de reconnaître l'anticorps monoclonal idiotypique;

b) les anticorps qu'elles sécrètent sont capables d'inhiber la fixation du ligand et/ou des agonistes et/ou des antagonistes dudit ligand;

c) les anticorps qu'elles sécrètent sont capables de reconnaître des cellules ou des constituants de cellules portant le récepteur dudit ligand.

L'invention concerne aussi un procédé de préparation de ces lignées cellulaires, les nouveaux anticorps monoclonaux sécrètés par ces cellules hybrides, et leur utilisation notamment comme réactifs marqueurs du récepteur du ligand ou comme ingrédients actifs dans la préparation de médicaments agonistes ou antagonistes dudit ligand.

ACTORUM AG

Nouvelles cellules hybrides sécrétant des anticorps anti-idiotypiques, nouveaux anticorps monoclonaux sécrétés par lesdites cellules hybrides, leur préparation et leur application.

La présente invention a pour objet de nouvelles cellules hybrides, les anticorps monoclonaux sécrétés par lesdites cellules hybrides, leur préparation et leur application.

On sait que les anticorps ou immunoglobulines sont antigéniques et que leur pouvoir immunogène révèle une hétérogénéité permettant de distinguer trois sortes de caractères antigéniques : les caractères isotypiques qui sont communs à toutes les immunoglobulines des individus d'une même espèce, les caractères allotypiques qui reflètent des variations génétiques à l'intérieur d'une même espèce et les caractères idiotypiques qui reflètent une spécificité propre à chaque clone cellulaire, sécrétant des immunoglobulines, d'un individu.

On sait aussi que les déterminants antigéniques idiotypiques sont liés à la conformation moléculaire propre à un site anticorps donné et sont donc situés sur la partie variable de la molécule d'immunoglobuline, c'est-à-dire sur le fragment Fab.

L'immunisation d'animaux avec des anticorps dirigés contre un haptène déterminé est donc susceptible de déclencher la production d'anticorps dirigés contre les déterminants antigéniques idiotypiques de l'anticorps anti-haptène. Toutefois, on obtient par cette technique des populations variées d'anticorps, et on a pu constater expérimentalement que les anticorps anti-idiotypiques dirigés contre la partie variable de l'anticorps anti-haptène sont eux-mêmes hétérogènes et possèdent des spécificités différentes.

Il était donc nécessaire de trouver des moyens de produire des anticorps anti-idiotypiques homogènes qui présentent un grand intérêt potentiel, notamment pour l'étude, la stimulation ou l'inhibition des récepteurs pharmacologiques.

On sait que le concept de récepteur est actuellement étendu à toute structure chimique de l'organisme capable de se lier à une molécule donnée. La molécule peut être exogène (c'est le cas en particulier d'un médicament) ou endogène (c'est le cas notamment d'une hormone ou d'un neuromédiateur). Les molécules capables de se lier avec un récepteur sont appelées d'une façon générale des "ligands".

Les anticorps anti-idiotypiques dirigés contre les déterminants idiotypiques d'anticorps anti-ligand sont susceptibles de reconnaître, tout comme le ligand, le récepteur dudit ligand.

On conçoit donc que les anticorps anti-idiotypiques sont susceptibles de jouer, par rapport au ligand, le rôle d'agoniste, c'est-à-dire de substance capable de se lier aux sites actifs des récepteurs du ligand et de les activer en provoquant les mêmes effets physiologiques que le ligand, ou le rôle d'antagoniste, c'est-à-dire de .susbstance qui se lie aux sites actifs des récepteurs du ligand sans qu'il en résulte d'effet physiologique.

Toutefois, l'effet des anticorps anti-idiotypiques sur les récepteurs ne peut être étudié avec certitude que si l'on dispose d'anticorps homogènes monospécifiques. Les techniques de préparation utilisées jusqu'à présent ne permettent pas d'obtenir en quantité importante des anticorps anti-récepteurs ayant des effets reproductibles, même lorsqu'il s'agit d'anticorps monoclonaux. C'est ainsi que parmi des centaines d'anticorps monoclonaux anti-récepteur cholinergique nicotinique qui ont été obtenus par James et al., FEBS Letters, 120, 145 (1980), un seul réagissait avec le site de liaison du récepteur.

La présente invention a notamment pour objet de nouvelles lignées cellulaires hybrides capables de sécréter des anticorps anti-idiotypiques dirigés contre un déterminant antigénique idiotypique d'une immunoglobuline anti-ligand, lesdits anticorps anti-idiotypiques étant capables de reconnaître sélectivement le récepteur dudit ligand.

Ces nouvelles cellules hybrides sont caractérisées par le fait qu'elles proviennent de la fusion d'une cellule lymphoïde capable de se multiplier in vitro avec des lymphocytes d'un animal immunisé contre l'immunoglobuline anti-ligand, que ladite immunoglobuline, contre laquelle l'animal donneur desdits lymphocytes a été immunisé, est une immunoglobuline monoclonale de provenance syngénique, et que lesdites cellules hybrides ont été sélectionnées successivement selon les critères cumulatifs suivants :

    a) Les anticorps qu'elles sécrètent sont capables de reconnaître l'anticorps monoclonal idiotypique ;

    b) Les anticorps qu'elles sécrètent sont capables d'inhiber la fixation du ligand et/ou des agonistes et/ou des antagonistes dudit ligand ;

    c) Les anticorps qu'elles sécrètent sont capables de reconnaître des cellules ou des constituants de cellules portant le récepteur dudit ligand.

L'invention a également pour objet les anticorps monoclonaux anti-idiotypiques sécrétés par les lignées cellulaires hybrides décrites ci-dessus.

L'invention a également pour objet un procédé de préparation desdites lignées cellulaires hybrides et/ou des anticorps monoclonaux anti-idiotypiques qu'elles sécrètent, caractérisé par le fait qu'il comporte les étapes suivantes :

a) on immunise un animal vertébré par administration de cellules syngéniques monoclonales fixées sécrétant ladite immunoglobuline anti-ligand ;

b) on fusionne, selon les techniques connues, des lymphocytes de l'animal immunisé avec des cellules lymphoïdes cultivables in vitro, en présence d'un promoteur de fusion ;

c) on sélectionne les cellules hybrides ainsi obtenues selon les critères successifs mentionnés précédemment ; et

d) si désiré, on cultive lesdites cellules hybrides sélectionnées et isole les anticorps anti-idiotypiques qu'elles sécrètent.

Dans des modes d'exécution particuliers, le procédé de l'invention peut encore présenter les caractéristiques qui vont être décrites ci-après, ces caractéristiques pouvant être prises isolément ou en combinaison.

L'immunisation est effectuée avec des cellules fixées, c'est-à-dire tuées avec un agent d'inactivation n'affectant pas sensiblement le pouvoir immunogène. On peut utiliser par exemple comme agent de fixation ou d'inactivation les agents habituellement utilisés pour la réalisation des vaccins avec des bactéries inactivées, notamment des agents chimiques comme le formol, le glutaraldéhyde, etc...

Les cellules utilisées comme immunogène sont notamment des cellules hybrides monoclonales obtenues selon les méthodes connues, par fusion de cellules lymphoïdes cultivables in vitro avec des lymphocytes d'animaux immunisés contre le ligand, puis sélection des cellules sécrétant des anticorps dirigés spécifiquement contre le ligand. L'immunisation est effectuée selon les techniques usuelles, par administration du ligand couplé à une macromolécule, en particulier à une protéine, de façon à le rendre immunogène.

De préférence, l'agent immunogène est administré par voie intraveineuse, par exemple à des souris, en l'absence d'adjuvant.

Une caractéristique de cette méthode d'immunisation est que l'on emploie comme agent d'immunisation, non pas l'immunoglobuline anti-ligand, mais la cellule productrice de cette immunoglobuline. On a en effet découvert que de telles cellules peuvent être utilisées avantageusement pour l'immunisation, car elles augmentent fortement la formation, par réponse immunitaire, d'anticorps dirigés contre les déterminants anti-idiotypiques. Ces cellules peuvent donc être utilisées comme porteur de l'antigène idiotypique.

L'immunisation des animaux, par exemple des souris, est réalisée de façon à provoquer une réponse immunitaire secondaire, selon les méthodes connues. On peut par exemple injecter l'immunogène par voie intraveineuse une fois par semaine pendant trois semaines avec environ $10^6$ à $10^9$ cellules

fixées, puis faire un rappel par voie intraveineuse au bout de 2 à 3 mois, trois jours avant la fusion.

Pour obtenir les cellules-mères utilisées dans l'opération de fusion cellulaire, on recueille des lymphocytes de l'animal immunisé. Par exemple, on prélève la rate des animaux immunisés et on recueille les splénocytes, selon les techniques usuelles.

On effectue ensuite l'étape de fusion des lymphocytes prélevés avec des cellules lymphoïdes monoclonales cultivables in vitro, par exemple des cellules de myélome de souris, de préférence des cellules non sécrétantes. Parmi les cellules de myélome de souris utilisables, on citera en particulier la lignée de cellules de myélome NS-1 gracieusement fournie par P. GOODFELLOW, (Imperial Cancer Research Fund, Londres). Cette lignée cellulaire, non sécrétante, a été sélectionnée notamment pour sa sensibilité à l'aminoptérine. Elle peut être cultivée par exemple sur un milieu RPMI 1640 (voir par exemple MOORE et al., Culture of Normal Human Leucocytes, J.A.M.A. 199, 519-524, 1967) contenant 10 % de sérum foetal de veau, 2 mM de glutamine, 1 mM de pyruvate de sodium, 100 UI/ml de pénicilline et 100 microgrammes/ml de streptomycine.

La fusion des cellules est une technique bien connue qui consiste essentiellement à mélanger des lymphocytes de l'animal immunisé et les cellules lymphoïdes monoclonales en présence d'un agent promoteur de fusion tel que le polyéthylèneglycol, selon les techniques connues, par exemple la technique de KOHLER et MILSTEIN, Nature, 256, 495-497 (1975).

Après incubation dans des conditions convenables, les cellules sont lavées et mises en culture sur un milieu sélectif permettant uniquement la croissance des cellules hybrides.

Un tel milieu sélectif peut être constitué par exemple par le milieu complet RPMI mentionné ci-dessus, auquel on a ajouté de l'hypoxanthine, de l'aminoptérine et de la thymidine. Il est connu que les cellules de myélome, qui sont dépourvues de l'enzyme hypoxanthine-guanine-phosphoribosyl-transférase ne se reproduisent pas sur les milieux contenant de l'hypoxantine, de l'aminoptérine et de la thymidine. Dans ces conditions, les cellules n'ayant pas fusionné ne peuvent pas se reproduire sur le milieu sélectif utilisé.

On recherche ensuite, après un temps de culture suffisant, la présence dans les surnageants de culture, des anticorps anti-idiotypiques selon les critères qui ont été indiqués ci-dessus. Pour mettre en oeuvre cette sélection, on utilise des méthodes immunologiques et classiques fondées sur la réaction antigène-anticorps. On sous-clone les cellules retenues à chaque étape des différents critères de sélection. Le sous-clonage est avantageusement effectué par la méthode des dilutions limites décrites par OI et

HERZENBERG, in : Selected Methods in Cellular Immunology, Eds. B.B. MISHELL et S.M. SHRIGI (Freeman, San Francisco) page 351, 1980.

Pour sélectionner les cellules hybrides sécrétant des anticorps anti-idiotypiques conformément aux critères énoncés ci-dessus, on peut par exemple utiliser les techniques qui sont décrites ci-après à titre d'illustration uniquement.

Pour vérifier que les anticorps anti-idiotypiques reconnaissent l'immunoglobuline anti-ligand, on peut fixer sur un support, par exemple sur une plaque de micro-titration, les cellules sécrétant ladite immunoglobuline qui ont servi d'agent d'immunisation.

On met en contact le support ainsi revêtu avec les surnageants de culture des cellules hybrides obtenues par l'opration de fusion décrite ci-dessus. Après incubation et lavage, on met alors en contact le support avec une solution contenant des anticorps anti-isotypiques marqués avec un agent traceur.

L'agent traceur est par exemple un marqueur enzymatique, radioactif ou fluorescent.

Par exemple, si les cellules hybrides produites sont des cellules murines, on peut utiliser comme anticorps isotypiques marqués des anticorps de lapin anti-(fragmentFc d'immunoglobulines de souris).

Pour rechercher la présence d'anticorps anti-idiotypiques reconnaissant l'immunoglobuline anti-ligand, on peut aussi utiliser un essai radio-immunologique direct à l'aide d'anticorps anti-idiotypiques marqués par voie biosynthétique.

Pour cela, on cultive les cellules hybrides obtenues par fusion dans un milieu contenant un agent nutritif marqué avec un isotope radioactif, par exemple de la méthionine marquée au soufre isotopique 35 ou encore de la leucine tritiée. Les surnageants de culture sont incubés sur des supports (plaques de microtitration) revêtus de l'immunoglobuline anti-ligand, puis après lavage, on procède au comptage du matériau radioactif éventuellement fixé sur le support.

Un essai radio-immunologique direct analogue, mais inversé, peut être effectué pour tester des fluides ascitiques formés après injection par voie péritonéale des cellules hybrides obtenues par fusion comme décrit ci-dessus. Après formation de l'ascite, on récolte le fluide ascitique, on le met en contact avec un support adsorbant, puis on met en contact le support ainsi revêtu des anticorps présents dans le fluide ascitique avec des surnageants de culture de la cellule ayant servi d'immunogène cultivée dans un milieu contenant un agent nutritif marqué avec un isotope radioactif, par exemple en présence de leucine tritiée.

Pour sélectionner les cellules hybrides dont les surnageants de culture contiennent des anticorps entrant en compétition avec le ligand (ou avec un agoniste ou un antigoniste dudit ligand) on fixe le ligand, éventuellement couplé à une macromolécule, sur un support adsorbant, et on ajoute un mélange de solutions en quantités équivalentes de surnageant de culture et d'immunoglobuline anti-ligand, lesdites immunoglobulines étant marquées avec un agent traceur. On sélectionne avec ce test les cellules hybrides produisant des anticorps capables d'inhiber la fixation des immunoglobulines anti-ligand sur le ligand.

Enfin, pour sélectionner les cellules hybrides capables de reconnaître spécifiquement le ligand, on peut fixer des cellules ou des constituants de cellules possédant le récepteur sur un support. On met en contact les surnageants de culture testés et on laisse incuber, puis, après lavage, on met en contact avec des anticorps anti-isotypiques marqués avec un agent traceur.

On peut aussi repérer les cellules hybrides sécrétant les anticorps anti-idiotypiques reconnaissant le récepteur à l'aide d'un test consistant à fixer sur un support adsorbant des cellules ou constituants de cellules possédant le marqueur. On met en contact le support ainsi revêtu avec les surnageants de culture testés, puis avec une solution d'anticorps anti-isotypiques marqués avec un agent traceur. On fait ensuite la même opération, mais avec l'étape supplémentaire consistant à mettre le support en contact d'abord avec une solution d'immunoglobulines anti-ligand avant de le mettre en contact avec la solution d'anticorps anti-isotypiques marqués. On évalue alors la quantité d'anticorps marqués éventuellement fixés sur le support de façon à déterminer si cette quantité a augmenté dans des proportions notables dans le cas où on a ajouté l'immunoglobuline anti-ligand. Si cela est bien le cas, cela prouve que les cellules hybrides produisent des anticorps qui sont capables à la fois de reconnaître le récepteur et l'immunoglobuline anti-ligand. En effet, dans ce cas, les immunoglobulines anti-ligand se fixent en quantité notable sur les anticorps anti-idiotypiques, et la quantité d'anticorps anti-isotypiques marqués qui se fixent augmente alors dans la même proportion.

Pour déterminer le type d'activité pharmacologique (agoniste ou antagoniste) des anticorps anti-idiotypiques de l'invention, on peut procéder soit directement, par l'étude de la réponse des organes effecteurs, soit indirectement, selon des méthodes connues, par exemple pour les récepteurs liés à l'adénylcyclase, par l'étude de l'effet de stimulation ou d'inhibition de l'adénycyclase par l'anticorps anti-idiotypique considéré.

L'invention s'étend aux anticorps dirigés contre un déterminant antigénique idiotypique d'une immunoglobuline anti-ligand monoclonale de provenance syngénique, caractérisés par le fait que lesdits anticorps sont des anticorps monoclonaux qui possèdent les propriétés cumulatives suivantes :

a) ils reconnaissent l'anticorps monoclonal idiotypique ;

b) ils sont capables d'inhiber la fixation du ligand et/ou des agonistes et/ou des antagonistes dudit ligand ;

c) ils sont capables de reconnaître des cellules ou des constituants de cellules portant le récepteur dudit ligand.

L'invention s'étend notamment aux anticorps monoclonaux anti-idiotypiques tels que définis précédemment, modifiés par marquage, par exemple avec un traceur radio-actif, fluorescent ou enzymatique, obtenus selon les méthodes classiques de préparation de tels anticorps marqués.

Par exemple, le traceur peut être un traceur radio-actif obtenu par addition chimique d'un isotope radio-actif tel que l'iode 125. En outre l'anticorps peut être marqué par voie bio-synthétique en cultivant les cellules hybrides productrices dans un milieu contenant des acides aminés marqués, par exemple la méthionine marquée au soufre 35, la leucine tritiée, etc...

Le couplage des anticorps avec un fluorochrome (par exemple l'iso-thiocyanate de fluorescéine ou de rhodamine) est également connu.

Le couplage des anticorps avec un traceur enzymatique est également connu en soi. Un enzyme est par exemple une peroxydase. L'activité enzymatique éventuellement présente sur le réactif après réalisation d'un test peut être déterminé selon les méthodes connues avec un substrat approprié permettant par exemple une révélation par colorimétrie, par fluorescence, par luminescence, par potentiométrie, etc...

L'invention s'étend en outre aux anticorps anti-idiotypiques, marqués ou non, tels que définis précédemment, fixés sur un support solide permettant leur utilisation comme agents immuno-absorbants dans des méthodes de chromatographie ou comme réactifs d'analyse.

Le support peut être réalisé avec tout matériau solide, biologique ou synthétique, doué de propriétés adsorbantes ou capables de fixer un agent de couplage. Ces matériaux sont connus et décrits dans la littérature. Parmi lesmatériaux solides capables de fixer les anticorps par adsorption, on citera par exemple le polystyrène, le polypropylène, les latex, etc... Parmi les matériaux utilisables pour fixer les anticorps par covalence à l'aide d'un

- 8 -

0180486

agent de couplage, on peut citer notamment le dextran, la cellulose, leurs dérivés aminés, etc...

Le support peut se présenter par exemple sous forme de disques, de tubes, de baguettes, de billes ou de plaques, en particulier de plaques de microtitration.

Les agents de couplage permettant de fixer par covalence les anticorps sur un support solide, sont des dérivés bi-fonctionnels tels que des dialdéhydes, des quinones, etc...

Les anticorps peuvent également être fixés sur des supports solides minéraux selon les méthodes décrites par exemple dans les brevets français n°2.319.399, 2.403.556 et 2.403.098.

L'invention s'étend également aux fragments d'anticorps anti-idiotypiques tels que définis précédemment, lesdits fragments comportant la partie variable de l'anticorps capable de reconnaître le récepteur. Il s'agit en particulier des fragments Fab qui peuvent être obtenus selon les méthodes connues, par exemple par un clivage enzymatique avec le papaïne ou des fragments $F(ab')_2$ obtenus par exemple par clivage enzymatique avec la pepsine, ou encore d'un fragment Fv.

Les anticorps de l'invention sont de préférence des IgG, des IgM ou des IgA.

L'invention s'étend également à l'utilisation des anticorps anti-idiotypiques monoclonaux comme agents de diagnostic, comme réactifs marqueurs du récepteur du ligand contre lequel sont dirigés les anticorps idiotypiques correspondants, comme réactifs d'étude du mécanisme d'action du ligand et/ou de ses agonistes ou antagonistes, et comme ingrédients actifs dans la préparation de médicaments agonistes ou antagonistes dudit ligand.

Par exemple, dans le domaine du diagnostique médical, les anticorps de l'invention pourront servir de marqueurs pour le récepteur et indiquer de cette manière la présence ou l'absence du récepteur sur les cellules ou les tissus cibles dans différentes conditions pathologiques.

Dans le domaine de la pharmacologie du récepteur, les anticorps ayant des caractéristiques d'agonistes d'une part, ou d'antagonistes d'autre part, peuvent être utilisés dans l'étude du mécanisme d'action du ligand endogène au niveau moléculaire.

Dans le domaine thérapeutique, les anticorps ou leur fragments $F(ab')_2$, Fab ou Fv peuvent être utilisés comme agents pharmacologiques ayant des propriétés de blocage ou de stimulation du récepteur, selon que les anticorps ont des propriétés d'agonistes ou d'antagonistes du ligand.

Les compositions pharmaceutiques contenant lesdits anticorps sont présentés sous les formes usuelles permettant l'administration par voie intramusculaire ou intraveineuse. Elles se présentent notamment sous la forme de solutions injectables ou de poudres lyophilisées permettant de reconstituer une solution injectable au moment de l'emploi.

La posologie varie notamment en fonction du poids corporel et de la maladie traitée. Par exemple, chez l'adulte, la dose quotidienne, par voie intraveineuse, peut varier de 50 mg à 1 g, en dose unique.

L'invention a également pour objet un dispositif destiné à l'utilisation des anticorps monoclonaux anti-idiotypiques comme réactifs de diagnostic ou d'étude des récepteurs ou des ligands. Ce dispositif est caractérisé par le fait qu'il comprend une ou plusieurs unités d'un support solide absorbant, un ou plusieurs conteneurs contenant l'anticorps monoclonal anti-idiotypique éventuellement marqué avec un agent traceur, et éventuellement un ou plusieurs conteneurs contenant l'anticorps anti-ligand marqué ou non, des cellules inactivées portant le récepteur, des tampons de dilution, d'incubation et de lavage, avec éventuellement un mode d'emploi, le tout étant réuni dans un même emballage à plusieurs compartiments constituant un coffret d'étude ou de diagnostic immunologique contenant, à titre de réactif principal, l'anticorps monoclonal anti-idiotypique de l'invention.

On va maintenant décrire un exemple de réalisation de l'invention en faisant référence plus particulièrement à l'obtention de lignées cellulaires hybrides sécrétant des anticorps monoclonaux anti-idiotypiques capables de reconnaître le récepteur bêta-adrénergique. Cette description de l'obtention des cellules hybrides capables de sécréter des anticorps anti-idiotypiques reconnaissant le récepteur bêta-adrénergique est faite à titre illustratif uniquement. Il est évident pour les spécialistes de l'immunologie que les procédés indiqués précédemment permettent d'obtenir d'une façon générale des anticorps monoclonaux anti-idiotypiques capables de reconnaître spécifiquement le récepteur d'un ligand, et de jouer un rôle d'agonistes ou d'antagonistes dudit ligand.

Par exemple, les anticorps anti-idiotypiques capables de reconnaître le récepteur $\beta_1$ ou $\beta_2$ auront, selon qu'ils ont des propriétés d'agonistes ou d'antagonistes, des effets $\beta$-stimulants ou $\beta$-bloquants correspondants.

EXEMPLE 1 - Préparation des anticorps anti-iditiotypiques

1° - Immunisation

L'immunogène est constitué par des cellules d'hybridomes monoclonales productrices d'anticorps anti-ligand bêta-adrénergique ; il s'agit des cellules d'hybridomes productrices de l'anticorps appelé 37 A 4, décrites par CHAMAT et al, J. of Immunology, Vol 133, P. 1547-1552 (1984). Ces cellules monoclonales appelées ci-après "cellules 37 A 4" ont été déposées à l'Institut PASTEUR le 17 Juillet 1984 sous le n° I.317.

On rappelle que l'anticorps 37 A 4 est un anticorps qui reconnait spécifiquement les antagonistes bêta-adrénergiques comme l'alprenolol ou les agonistes bêta-adrénergiques tel que l'isoprotérenol.

Pour être utilisées comme immunogènes, les cellules 37 A 4 sont tuées par traitement avec un agent usuel de fixation tel que le paraformaldéhyde ou le glutaraldéhyde, de façon à empêcher toute multiplication de la cellule tout en conservant le pouvoir immunogène. On peut traiter par exemple les cellules de 37 A 4 pendant 15 minutes avec une solution à 1 % de paraformaldéhyde en tampon PBS de pH 7,4. On inactive ensuite les groupements aldéhydes n'ayant pas réagit par mise en contact pendant 2 heures avec une solution 0,2 M de glycine. Les cellules fixées sont lavées deux fois avec du tampon PBS et sont conservées à 4°C en tampon PBS contenant un agent bactériostatique (azidure de sodium ou merthiolate par exemple). Dans le cas présent, on a conservé les cellules fixées avec du tampon PBS contenant 0,02 % d'azidure de sodium.

On immunise des souris balb/c âgées de 8 semaines en injectant par voie intraveineuse, une fois par semaine pendant 3 semaines, $10^7$ cellules fixées, lavées avec du tampon PBS ne contenant pas d'azidure. Après une période de repos de 2 mois on effectue un rappel par voie intraveineuse avec la même quantité de cellules 3 jours avant l'opération de fusion.

2° - Fusion

On prépare une suspension de cellules spléniques individualisées par injection de milieu RPMI dans la rate isolée des souris immunisées. On lave les cellules obtenues et les mélange avec des cellules de myélome NS 1.

Après centrifugation, on élimine le surnageant et ajoute 2 ml d'une solution à 41 % de polyéthylèneglycol 1500 (Merck RFA) au culot de cellules en agitant modérément à 37°C. Après une minute, on ajoute progressivement 35 ml de milieu RPMI à la suspension. Les cellules fusionnées sont centrifugées, remises en suspension dans 50 ml de milieu RPMI contenant 10 % de sérum de veau foetal inactivé par la chaleur, puis elles sont réparties à raison de 2 x $10^6$ cellules/puits dans 2 plaques (NUNC) à 24 puits.

Les cellules sont cultivées à 37°C dans un incubateur humidifié en atmosphère contenant 10 % de $CO_2$. Après 24 heures, on ajoute à chaque puits 1 ml de milieu RPMI contenant 10 % de sérum de veau foetal, 0,1 mM d'hypoxanthine, 0,5 M d'aminoptérine et 0,1 M de thymidine.

Au 4e jour puis au 6e jour de culture, on retire 1 ml du milieu et le remplace par 1 ml du même milieu nouvellement préparé. On détecte les hybridomes sécrétant les anticorps anti-idiotypiques en soumettant les surnageants aux tests suivants.

3° - Essais immunoenzymatiques

On revêt des plaques de microtitration (DYNATECH) avec $3 \times 10^5$ cellules 37 A 4 par puits. Pour cela, on effectue d'abord un pré-revêtement avec une solution de polylysine à 10 microgrammes/ml pendant 2 heures à 37°C. Les cellules sont ensuite centrifugées pendant 40 minutes à 1000 g, les cellules sont fixées dans le glutaraldéhyde à 0,25 %, incubées pendant 1 heure en solution de glycine 0,2 M et le glutaraldéhyde en excès est inactivé par addition d'un tampon PBS -gélatine 0,25 % - Tween 0,1 % (tampon P.G.T.) pendant 2 heures à 37°C.

On transfère sur chaque puits 100 microlitres de surnageant des cellules d'hybridome préparées au stade précédent et on laisse incuber pendant une nuit à 4°C. Après plusieurs lavages en milieu P.G.T., on ajoute en deux heures à 37°C, 50 microlitres d'une dilution au millième de sérum de lapin anti-(fragments Fc de souris) conjugué à la peroxydase.

Après lavage des plaques, on ajoute 200 microlitres de substrat (eau oxygénée-ABTS) dans chaque puits. On laisse la réaction enzymatique se développer pendant 30 minutes puis on l'arrête par addition de 50 microlitres de SDS (dodécylsulfate de sodium) à 10 %. On lit les résultats sur un appareil ARTEK ELISA à 405 nm.

Note : ABTS est une abréviation désignant l'acide 2,2'-azino-di(3-éthylbenzothiazoline) sulfonique.

4° - Essais radio-immunologiques directs à l'aide d'anticorps anti-idiotypiques marqués par voie biosynthétique

On cultive des hybridomes secrétant les anticorps anti-idiotypiques, détectés grâce au test immuno-enzymatique précédent, dans des plaques de cultures à 24 puits dans lesquels on a ajouté au milieu de culture 0,5 Ci/puits de méthionine marquée au $^{35}$S. Après 16 heures de culture, on teste les surnageants en les faisant incuber une nuit à 4°C, à raison de 10 microgrammes /ml, sur des plaques de microtitration de polychlorure de vinyle (DYNATECH) revêtues d'anticorps 37 A4, pendant une nuit à 4°C.

Après plusieurs lavages, le fond de chaque puits est découpé et transféré dans une cuve à scintillation puis traité avec 0,2 ml d'une solution d'acide chlorhydrique 1N et 3 ml de Biofluor (NEN) puis on compte la radioactivité dans un compteur (INTERTECHNIQUE FRANCE).

On utilise également le même essai radio-immunologique direct pour tester les fluides ascitiques provenant d'ascites provoquées par injection à des souris des hybridomes préparés tels que précédemment. Les fluides ascitiques sont adsorbés sur des plaques de filtration de nitrocellulose Millititer HA 96 (MILLIPORE Co.) pendant 4 heures à température ambiante. Les plaques sont lavées en milieu P.G.T. et mises à incuber avec les surnageants

0180486

de culture d'hybridomes sécrétant l'anticorps 37 A 4 marqué par voie bio-synthétique à la leucine tritiée selon la méthode décrite par GUILLET et al., Mol. Immunol. 20, 1059 (1983).

5° - Essais radio-immunologiques : Tests d'inhibition

On utilise des anticorps 37 A 4 marqués par voie biosynthétique à la leucine tritiée, comme indiqué ci-dessus.

On titre ces anticorps sur des plaques de polychlorure de vinyle revêtues avec 50 microlitres/ml d'un conjugué alprénolol-hémocyanine de Megathure crenulata (commercialisée par Calbiochem - USA) dans un tampon glycine 0,1 M de pH 2,8 et on sature les sites non revêtus avec du P.G.T.

Les anticorps 37 A 4 utilisés ici sont des anticorps marqués par voie biosynthétique à la leucine tritiée, comme indiqué précédemment. On prépare par ailleurs des volumes égaux de surnageants de culture d'hybridomes obtenus précédemment et de solution d'anticorps 37 A 4 donnant 50 % de la réponse maximum. On laisse incuber ce mélange pendant une nuit à 4°C puis on le transfère sur les plaques revêtues de conjugés alprénolol-hémocyanine, à raison de 10 microgrammes/ml par puits et on laisse incuber pendant 2 heures à 37°C. Les puits sont ensuite lavés et on procède au comptage de la radioactivité comme indiqué précédemment.

6° - Essais immunoenzymatiques sur récepteurs

On utilise dans ce test des plaques revêtues de cellules possédant un grand nombre de bêta récepteurs, en particulier les cellules d'épidermes humains A 431 (DELAVIER-KLUTCHKO et al., Febs Lett. 169, 151-155 (1984) ), ou bien des plaques revêtues de membranes de mastocytomes de souris P 815 (HOEBEKE et al., Adv. Cyclic. Nucl. Res. 17 73-80 (1984) ), ou encore des érythrocytes de dindon (ATLAS et al., Proc. Natl. Acad. Sci. USA 71, 4246.

Pour comparaison, on utilise également des plaques revêtues de cellules lymphoïdes de lymphocytes B de lapin TRSC-1 (KOSKAS et al., Eur. J. Immunol. 11 388-392 (1984) ) qui ne possèdent pas de bêta-récepteur.

On fixe les cellules ou les membranes avec du glutaraldéhyde à 0,25 % et on sature les puits avec du P.G.T.

Les cellules A 431 sont cultivées sur des plaques de 96 puits (LINBRO) et les membranes de mastocytomes et les érythrocytes de dindon sont absorbés sur des plaques de polystyrène (ANDRE et al, EMBO J., 3, 17 (1984).

On effectue sur les plaques ainsi revêtues le test immunoenzymatique décrit précédemment, c'est-à-dire qu'on ajoute d'abord les surnageants de culture d'hybridomes obtenus ci-dessus puis on ajoute les anticorps de lapins marqués anti-fragment Fc de souris .

7° - Test immunoenzymatiques avec les récepteurs

On utilise des plaques dont les puits sont revêtus avec une préculture de cellules A 431. On ajoute aux plaques ainsi revêtues des surnageants de culture des hybridomes obtenus précédemment. On lave et met ensuite en contact avec une solution contenant des anticorps 37 A 4. On met enfin en contact avec des anticorps de lapins anti-gammaglobulines (H + L) de souris conjugués à la peroxydase à une dilution au millième qui ne donne pas de réponse en présence des cellules A 431 revêtues seulement des anticorps anti-idiotypiques (sans addition d'anticorps 37 A 4). L'augmentation de la réponse après mise en contact avec l'anticorps 37 A 4 permet de mesurer la quantité d'anticorps idiotypiques fixée sur les sites de liaison libres des anticorps anti-idiotypiques liés au bêta-récepteur.

8° - Résultats

Pour obtenir des hybridomes sécrétant des anticorps anti-idiotypiques dirigés contre l'anticorps monoclonal 37 A 4, lui-même dirigé contre les ligands bêta-adrénergiques, on a tiré avantage de la présence de l'anticorps sur la surface de l'hybridome sécrétant l'anticorps 37 A 4. Les résultats obtenus montrent que l'anticorps de membrane expose seulement la fraction Fab à la surface de la cellule, comme cela a d'ailleurs été vérifié directement. L'immunisation des souris avec l'hybridome sécrétant l'anticorps anti-37 A 4 plutôt qu'avec l'anticorps lui-même a permis d'obtenir après fusion une forte proportion d'anticorps anti-idiotypiques. Cela permet également de réaliser le premier test de sélection des anticorps anti-idiotypiques en revêtant des plaques de micro-titration non pas avec l'anticorps 37 A 4 mais avec la cellule productrice : Dans ces conditions, seuls les surnageants des hybridomes sécrétant des anticorps anti-idiotypiques donnent une réponse accrue avec les anticorps anti-Fc de souris conjugués à la peroxydase. On a trouvé que 50 % des hybridomes obtenus donnaient un résultat positif sur ce test.

Sur 24 clones positifs dans ce premier test, 6 ont donné une réaction positive dans le test d'inhibition de la fixation de l'alprenolol.

Les essais radio-immunologiques direct et inversé permettent de sélectionner rapidement les hybridomes sécrétant l'anti-idiotype pour le sous-clonage.

Enfin, sur 6 hybridomes, 3 ont donné des résultats positifs dans l'essai immuno-enzymatique sur récepteur.

L'une de ces lignées cellulaires d'hybridomes sécrétant des anticorps anti-idiotypiques capables de reconnaître le récepteur bêta-adrénergique, l'IgM "Ab2B4" a été déposé à l'Institut PASTEUR le 17 Juillet 1984 sous le n°I-318 L'étude de l'anticorps Ab2B4 a permis de montrer qu'il

0180486

reconnaît sur les protéines de membrane des cellules A 431 un polypeptide ayant un poids moléculaire de 52 kD, de façon spécifique. Comme il est connu que le récepteur bêta-adrénergique présent sur les cellules A 431 est un récepteur $\beta 2$ et que le poids moléculaire des récepteurs $\beta 2$ humains est d'environ 56 kD, cela confirme que l'anticorps Ab2B4 reconnaît effectivement le récepteur bêta-adrénergique des cellules A 431.

On a également observé par test ELISA que l'anticorps Ab2B4 reconnaît des sites ayant des structures communes à la fois aux récepteurs bêta-adrénergique et à l'anticorps 37 A 4.

On a également démontré la reconnaissance spécifique des récepteurs bêta-adrénergiques des cellules A 431 par l'anticorps Ab2B4 par immuno-fluorescence. En effet, des cellules traitées avec l'antagoniste alprénolol présentent une immuno-fluorescence spécifique tandis que des cellules prétraitées avec l'agoniste 1-isoproterenol ne présentent pas de fluorescence.

Enfin, l'anticorps Ab2B4 présente des propriétés de stimulation de la production d'AMP cyclique. Cela montre que l'anticorps Ab2B4 a des propriétés stimulantes, autrement dit des propriétés d'agoniste. L'effet de l'anticorps Ab2B4 est comparable à celui de l'iso-proterenol $10^{-9}$M. Il en résulte que sur une base moléculaire, la molécule d'Ab2B4 est 25 fois moins active que l'agoniste 1-isoproterenol dans l'induction de l'accumulation d'AMP cyclique.

En conclusion, l'anticorps Ab2B4 n'est pas seulement un réactif commode pour la visualisation des récepteurs bêta-adrénergiques et pour la caractérisation de leur structure, mais il possède aussi une activité physiologique intrinsèque d'agoniste bêta-adrénergique.

Les indications thérapeutiques, ainsi que les effets secondaires, de l'anticorps Ab2B4 sont celles des $\beta_2$- stimulants.

0180486

REVENDICATIONS

1. Lignées cellulaires hybrides capables de sécréter des anticorps anti-idiotypiques dirigés contre un déterminant antigénique idiotypique d'une immunoglobuline anti-ligand, caractérisées par le fait qu'elles proviennent de la fusion d'une cellule lymphoïde capable de se multiplier in vitro avec des lymphocytes d'un animal immunisé contre l'immunoglobuline anti-ligand, que ladite immunoglobuline, contre laquelle l'animal donneur desdits lymphocytes a été immunisé, est une immunoglobuline monoclonale de provenance syngénique, et que lesdites cellules hybrides ont été sélectionnées successivement selon les critères cumulatifs suivants :

a) Les anticorps qu'elles sécrètent sont capables de reconnaître l'anticorps monoclonal idiotypique ;

b) Les anticorps qu'elles sécrètent sont capables d'inhiber la fixation du ligand et/ou des agonistes et/ou des antagonistes dudit ligand ;

c) Les anticorps qu'elles sécrètent sont capables de reconnaître des cellules ou des constituants de cellules portant le récepteur dudit ligand.

2. Lignées cellulaires selon la revendication 1, caractérisées par le fait qu'elles sécrètent des IgG, des IgM, ou des IgA.

3. Lignées cellulaires selon l'une quelconque des revendications précédentes, caractérisées par le fait qu'elles sécrètent des anticorps capables de reconnaître les récepteurs bêta-adrénergiques.

4. Lignée cellulaire, selon la revendication 1, sécrétant des anticorps capables de reconnaître les récepteurs bêta-adrénergiques, déposée à l'Institut Pasteur le 17 Juillet 1984 sous le n°I-318.

5. Procédé de préparation des lignées cellulaires hybrides et/ou des anticorps monoclonaux anti-idiotypiques qu'elles sécrètent, caractérisé par le fait qu'il comporte les étapes suivantes :

a) on immunise un animal vertébré par administration de cellules syngéniques monoclonales fixées sécrétant ladite immunoglobuline anti-ligand ;

b) on fusionne, selon les techniques connues, les lymphocytes de l'animal immunisé avec des cellules lymphoïdes cultivables in vitro, en présence d'un promoteur de fusion ;

c) on sélectionne les cellules hybrides ainsi obtenues selon les critères successifs mentionnés précédemment ; et

d) si désiré, on cultive lesdites cellules hybrides sélectionnées et isole les anticorps anti-idiotypiques qu'elles sécrètent.

6. Procédé selon la revendication 5, caractérisé par le fait que l'on effectue l'immunisation de façon à obtenir par réaction immunitaire des lymphocytes sécrétant des IgG, des IgM ou des IgA.

7. Procédé selon l'une quelconque des revendications 5 et 6, caractérisé par le fait que l'on effectue l'immunisation par voie intraveineuse en l'absence d'adjuvant.

8. Procédé selon l'une quelconque des revendications 5 à 7, caractérisé par le fait que l'immunisation est effectuée avec des cellules monoclonales sécrétant des immunoglobulines dirigées contre un ligand des récepteurs bêta-adrénergiques.

9. Anticorps, ou fragments d'anticorps, dirigés contre un déterminant antigénique idiotypique d'une immunoglobuline anti-ligand monoclonale de provenance syngénique, caractérisés par le fait que lesdits anticorps sont des anticorps monoclonaux qui possèdent les propriétés cumulatives suivantes :

    a) ils reconnaissent l'anticorps monoclonal idiotypique ;

    b) ils sont capables d'inhiber la fixation du ligand et/ou des agonistes et/ou des antagonistes dudit ligand ;

    c) ils sont capables de reconnaître des cellules ou des constituants de cellules portant le récepteur dudit ligand.

10. Anticorps anti-idiotypiques monoclonaux selon la revendication 9, caractérisés par le fait qu'ils reconnaissent les récepteurs bêta-adrénergiques.

11. Anticorps selon l'une quelconque des revendications 9 et 10, caractérisés par le fait que par fixation sur le récepteur, ils stimulent la production d'AMP cyclique.

12. Anticorps anti-idiotypique monoclonaux, caractérisés par le fait qu'ils sont sécrétés par la cellule hybride "Ab2B4", déposée à l'Institut PASTEUR le 17 Juillet 1984 sous le n° I-318.

13. Anticorps selon l'une quelconque des revendications 9 à 12, caractérisés par le fait qu'ils sont modifiés par marquage avec un traceur ou par clivage enzymatique.

14. Utilisation des anticorps tels que définis dans l'une quelconque des revendications 9 à 13, comme agents de diagnostic, comme réactifs marqueurs du récepteur du ligand contre lequel sont dirigés les anticorps idiotypiques correspondants, comme réactifs d'étude du mécanisme d'action du ligand et/ou de ses agonistes ou antagonistes, ou comme ingrédients actifs dans la préparation de médicaments agonistes ou antagonistes dudit ligand.

15. Composition pharmaceutique, caractérisée par le fait qu'elle renferme comme ingrédient actif un anticorps monoclonal anti-idiotypique tel que défini dans l'une quelconque des revendications 9 à 14.

16. Dispositif destiné à l'utilisation des anticorps monoclonaux anti-idiotypiques comme réactif de diagnostic ou d'étude des récepteurs ou des ligands, caractérisé par le fait qu'il comprend une ou plusieurs unités d'un support solide absorbant, un ou plusieurs conteneurs contenant l'anticorps monoclonal anti-idiotypique éventuellement marqué avec un agent traceur, et éventuellement un ou plusieurs conteneurs contenant l'anticorps anti-ligand marqué ou non, des cellules inactivées portant le récepteur, des tampons de dilution, d'incubation et de lavage, avec éventuellement un mode d'emploi, le tout étant réuni dans un même emballage à plusieurs compartiments.

Office européen
des brevets

**RAPPORT DE RECHERCHE EUROPEENNE**

## DOCUMENTS CONSIDERES COMME PERTINENTS

| Catégorie | Citation du document avec indication, en cas de besoin, des parties pertinentes | Revendication concernee | CLASSEMENT DE LA DEMANDE Int Cl 4) |
|---|---|---|---|
| X | PROC. NATL. ACAD. SCI. USA, vol. 81, janvier 1984, pages 272-276; J. CRAIG VENTER et al.: "Monoclonal antibodies detect the conservation of muscarinic cholinergic receptor structure from Drosophila to human brain and detect possible structural homology with alpha1-adrenergic receptors" * Page 272, colonne de droite, ligne 3 avant la fin à page 273, colonne de droite, ligne 19 * | 1-14 | C 12 N    5/00 C 12 P   21/00 G 01 N   33/577 A 61 K   39/395 |
| X | BIOLOGICAL ABSTRACTS/RRM, vol. 28, 1985, réf. no. 98313; D.M. CHUANG: "A monoclonal antibody to a membrane component that interacts with beta-adrenergic receptor site" & SOCIETY FOR NEUROSCIENCE ABSTRACTS(US) 1984, vol. 10, no. 2, p1100 * Abrégé * | 9-13 | |
| X | BIOLOGICAL ABSTRACTS, vol. 79, no. 6, 1985, page AB-480, réf. no. 50027; J.-G. GUILLET et al.: "Production and detection of monoclonal anti-idiotype antibodies directed against a monoclonal anti-beta-adrenergic ligand antibody" & J. IMMUNOL. METHODS 74(1): 163-172, 1984 * Abrégé * | 1-16 | |

DOMAINES TECHNIQUES RECHERCHES (Int. Cl 4)

A 61 K
C 12 P

-/-

Le présent rapport de recherche a été établi pour toutes les revendications

| Lieu de la recherche | Date d'achèvement de la recherche | Examinateur |
|---|---|---|
| LA HAYE | 11-11-1985 | REMPP G.L.E. |

Office européen
des brevets

| | DOCUMENTS CONSIDERES COMME PERTINENTS | | Page 2 |
|---|---|---|---|
| Catégorie | Citation du document avec indication, en cas de besoin, des parties pertinentes | Revendication concernee | CLASSEMENT DE LA DEMANDE (Int. Cl 4) |
| X,P | PROC. NATL. ACAD. SCI. USA, vol. 82, mars 1985, pages 1781-1784; G. GUILLET et al.: "Beta-adrenergic agonist activity of a monoclonal anti-idiotypic antibody" * Pages 1781 et 1782 "Materials and methods" * | 1-14 | |
| | --- | | |
| X,P | THE JOURNAL OF IMMUNOLOGY, vol. 133, no. 3, septembre 1984, pages 1547-1552, The American Association of immunologists, US; SOULAIMA CHAMAT et al.: "Monoclonal antibodies specific for beta-adrenergic ligands" * Pages 1547-1548, "Materials and Methods" * | 1-14 | |
| | --- | | |
| X,P | CHEMICAL ABSTRACTS, vol. 103, no. 13, 30 septembre 1985, page 108, réf. no. 99220b, Columbus, Ohio, US; CHUANG, DE MAW: "A monoclonal antibody to a membrane component that interacts with the beta-adrenergic receptor" & J. CYCLIC NUCLEOTIDE PROTEIN PHOSPHORYLATION RES. 1985, 10(3), 281-92 * Abrégé * | 9-14 | DOMAINES TECHNIQUES RECHERCHES (Int. Cl 4) |
| | --- -/- | | |

Le présent rapport de recherche a été établi pour toutes les revendications

| Lieu de la recherche | Date d'achèvement de la recherche | Examinateur |
|---|---|---|
| LA HAYE | 11-11-1985 | REMPP G.L.E. |

**Office européen des brevets**

# RAPPORT DE RECHERCHE EUROPEENNE

| DOCUMENTS CONSIDERES COMME PERTINENTS | | | Page 3 |
|---|---|---|---|

| Catégorie | Citation du document avec indication, en cas de besoin, des parties pertinentes | Revendication concernee | CLASSEMENT DE LA DEMANDE Int Cl 4 |
|---|---|---|---|
| Y | CHEMICAL ABSTRACTS, vol. 94, 1981, page 389, réf. no. 135520f, Columbus, Ohio, US; C.M. FRASER et al.: "Monoclonal antibodies to beta-adrenergic receptors: Use in purification and molecular characterization of beta-receptors" * Abrégé * | 1,9 | |
| Y | BIOLOGICAL ABSTRACTS, vol. 76, no. 8, 1983, page 6656, réf. no. 60960; C.M. FRASER et al.: "The size of the mammalian lung beta2-adrenergic receptor as determined by target size analysis and immunoaffinity chromatography" * Abrégé * | 1,9 | |
| | | | DOMAINES TECHNIQUES RECHERCHES (Int. Cl.4) |
| A | EP-A-0 079 466 (MAX-PLANCK-GESELLSCHAFT ZUR FÖRDERUNG DER WISSENSCHAFTEN e.V.) | | |

Le présent rapport de recherche a été établi pour toutes les revendications

| Lieu de la recherche | Date d'achèvement de la recherche | Examinateur |
|---|---|---|
| LA HAYE | 11-11-1985 | REMPP G.L.E. |